# EUROPEAN PATENT APPLICATION

(11) **EP 3 061 537 A1**
(43) Date of publication of application: **31.08.2016**
(21) Application number: 14855468.6
(22) Date of filing: 17.09.2014
(51) Int. Cl.: B21D 5/01, A61B 17/68

(54) **BENDING METHOD AND BENDING DEVICE**

(30) Priority: 23.10.2013 JP 2013220028
(71) Applicant: Olympus Corporation, Hachioji-shi Tokyo 192-8507 (JP)
(72) Inventor: TANIGUCHI, Hirofumi, Tokyo 192-8507 (JP)
(74) Representative: Gunzelmann, Rainer
(86) International application number: PCT/JP2014/074473
(87) International publication number: WO 2015/060041

(57) **Abstract**

The possibility of performing a bending process again on an area that has already been bent once is reduced. Provided is a bending device (1) including two receiving sections (8) disposed with a distance therebetween; a pressing section (9) that presses a plate-like workpiece, which is supported in a bridged manner between the receiving sections (8), into a space between the receiving sections (8); an operable section (5, 6) that applies an external force for causing the pressing section (9) to generate a pressing force; and a marker retaining section (10) that is attached to at least one of the pressing section (9) and the receiving section (8) side and that retains a marker to be adhered to a pressed area of whichever one of surfaces of the workpiece by means of the pressing force.

## Description

### {Technical Field}

The present invention relates to bending methods and bending devices.

### {Background Art}

In the related art, a known processing tool includes two receiving protrusions spaced apart from each other such that an osteosynthesis plate can be bridged therebetween, and a pressing section that moves toward an area between the receiving protrusions. The osteosynthesis plate supported between the receiving protrusions is bent by sandwiching the osteosynthesis plate between the pressing section and the receiving protrusions and pressing against the osteosynthesis plate using the pressing section (for example, see Patent Literature 1).

### {Citation List}

### {Patent Literature}

{PTL 1}
Publication of Japanese Patent No. 4071563

### {Summary of Invention}

### {Technical Problem}

However, the material of the osteosynthesis plate expected to be used with the processing tool in Patent Literature 1 is a material with high toughness, such as stainless steel or a titanium alloy. In a case where a highly fragile material, such as magnesium or a magnesium alloy, is used as the osteosynthesis plate, if the bending process is performed again on an area that has already been bent once, there is a problem in that the osteosynthesis plate may easily break due to repeated plastic deformation. In particular, for example, if the same area is bent in the reverse direction after a fine bending process that is difficult to visually recognize, the material may break, which is problematic in that it is difficult for a physician to perform the bending process for an osteosynthesis treatment in an operating room.

The present invention has been made in view of the circumstances described above, and an object thereof is to provide a bending method and a bending device that can reduce the possibility of performing a bending process again on an area that has already been bent once.

### {Solution to Problem}

In order to achieve the aforementioned object, the present invention provides the following solutions.

An aspect of the present invention provides a bending method including a setting step for setting a plate-like workpiece in a bridged manner between two receiving sections disposed with a distance therebetween, and a pressing step for causing a pressing force to act on a pressing section so as to press the workpiece set between the receiving sections into a space between the receiving sections. The pressing step includes causing a marker to adhere to a pressed area of whichever one of surfaces of the workpiece. The marker indicates that the area has been pressed.

According to this aspect, when the plate-like workpiece is supported by the two receiving sections so as to bridge between the two receiving sections in the setting step and the pressing section is moved toward the space between the receiving sections in the pressing step, the workpiece is pressed by the pressing section, and a bending process for the workpiece thus commences. When a pressing force is applied from the pressing section to the workpiece disposed between the pressing section and the receiving sections, the marker adheres to the pressed area of whichever one of the surfaces of the workpiece. Thus, an operator can recognize that the area has already been bent once based on the adhered marker even if the bending of the workpiece is too slight to be visually recognized. Then, by checking the marker, the operator can readily prevent another bending process from being performed again on the area to which the marker is adhered.

In the above aspect, the pressing step may include disposing the marker at the pressing section side of the workpiece and causing the marker to adhere to the surface of the workpiece by means of the pressing force.

Accordingly, since the marker adheres to the surface of the workpiece pressed by the pressing section, the bent position can be confirmed in the surface that is recessed after the bending process. In this case, the meaning of the expression "disposing the marker at the pressing section side of the workpiece" includes disposing a marker carrying member, such as a sheet, at a position interposed between the pressing section and the workpiece, in addition to allowing the pressing section used for performing the bending process to retain the marker. In either case, the marker indicating the bent area can be adhered to the surface of the workpiece.

In the above aspect, the pressing step may include pressing against the workpiece by using the pressing section, which retains the marker on a surface thereof at the workpiece side.

Accordingly, the marker can be adhered to the workpiece by simply sandwiching the workpiece between the pressing section and the receiving sections and pressing against the workpiece, thereby allowing for a simple bending process.

In the above aspect, the pressing step may include disposing the marker at the receiving section side of the workpiece and causing the marker to adhere to the surface of the workpiece by means of the pressing force.

Accordingly, because the marker adheres to the surface at the receiving section side of the workpiece, the bent position can be confirmed in the surface that becomes a protruding side after the bending process. In this case, the meaning of the expression "disposing the marker at the receiving section side of the workpiece" includes disposing a marker carrying member, such as a sheet, at a position interposed between the receiving sections and the workpiece, in addition to allowing the receiving sections used for performing the bending process to retain the marker. In either case, the marker indicating the bent area can be adhered to the surface of the workpiece.

In the above aspect, the workpiece may be an osteosynthesis plate, and the marker may be composed of a biocompatible material.

Accordingly, it is possible to manufacture an osteosynthesis plate that can be applied to bones without having to remove the marker after performing the bending process.

Another aspect of the present invention provides a bending device including two receiving sections disposed with a distance therebetween; a pressing section that presses a plate-like workpiece, which is supported in a bridged manner between the receiving sections, toward an area between the receiving sections; an operable section that applies an external force for causing the pressing section to generate a pressing force; and a marker retaining section that is attached to at least one of the pressing section and the receiving section side and that retains a marker to be adhered to a pressed area of whichever one of surfaces of the workpiece by means of the pressing force.

According to this aspect, when the plate-like workpiece is supported by the two receiving sections so as to bridge between the two receiving sections and an external force is applied to the pressing section, the workpiece is pressed by the pressing section toward the space between the receiving sections, and a bending process for the workpiece thus commences. As the external force applied to the manual operable section is increased, the pressing force increases, so as to cause the material constituting the workpiece to exceed the elastic range and enter the plastic range, whereby the workpiece plastically deforms.

In this case, when the pressing force acts on the workpiece from the pressing section or the receiving section side, the marker retained in the marker retaining section attached to at least one of the pressing section and the receiving section side adheres to the pressed area of whichever one of the surfaces of the workpiece. Thus, even when a fine bending process is performed, the bent position can be identified based on the adhered marker, so that a warning can be given to prevent a bending process from being performed again on an area that has already been bent once.

In the above aspect, the marker retaining section may be detachably attached to at least one of the pressing section and the receiving section side.

Accordingly, in a case where the marker retained in the marker retaining section decreases as a result of performing the bending process, the marker retaining section may be detached and be replaced with a new marker retaining section, so that the bending process can be continuously performed while causing the marker to adhere to the bent area.

In the above aspect, the marker may be formed of powder or liquid, and the marker retaining section may have a porous structure that is capable of retaining the marker within pores.

Accordingly, the marker can be readily retained within the pores in the porous marker retaining section, so that the marker formed of powder or liquid can be readily transferred onto the surface of the workpiece when a pressing force is applied.

In the above aspect, the marker retaining section may be attached to a surface at the receiving-section side of the pressing section.

Accordingly, since the marker adheres to the surface of the workpiece to be pressed by the pressing section, the bent position can be confirmed in the surface that is recessed after the bending process.

In the above aspect, the marker retaining section may be attached between the two receiving sections.

Accordingly, because the marker adheres to the surface at the receiving section side of the workpiece, the bent position can be confirmed in the surface that becomes a protruding side after the bending process.

In the above aspect, the workpiece may be an osteosynthesis plate, and the marker may be composed of a biocompatible material.

Accordingly, it is possible to process an osteosynthesis plate that can be applied to bones without having to remove the marker after performing the bending process.

### {Advantageous Effects of Invention}

The present invention is advantageous in that the possibility of performing a bending process again on an area that has already been bent once can be reduced.

### {Brief Description of Drawings}

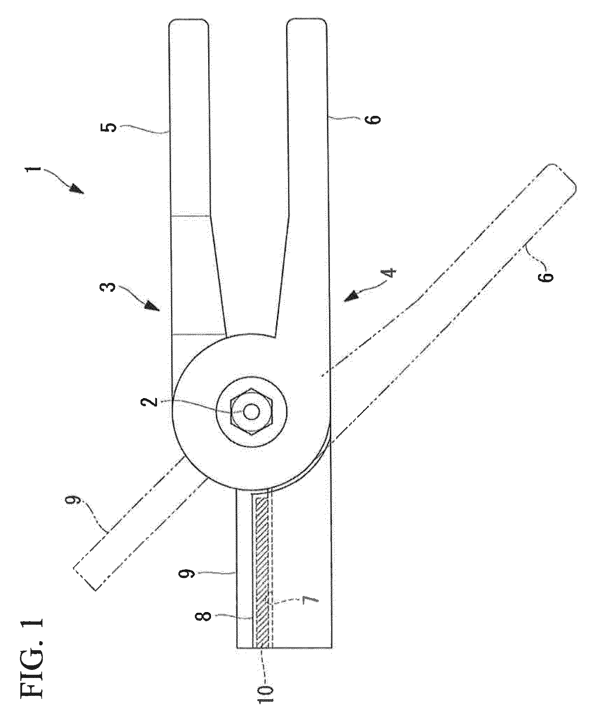

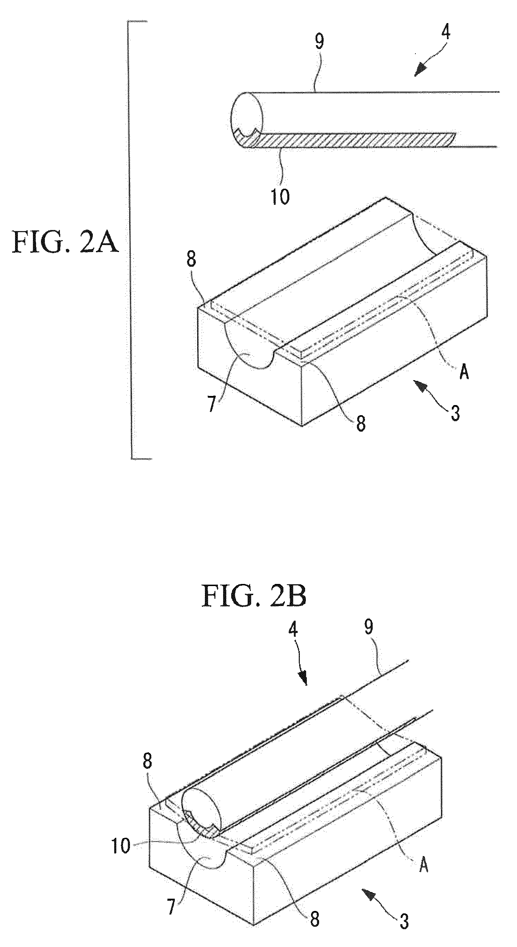

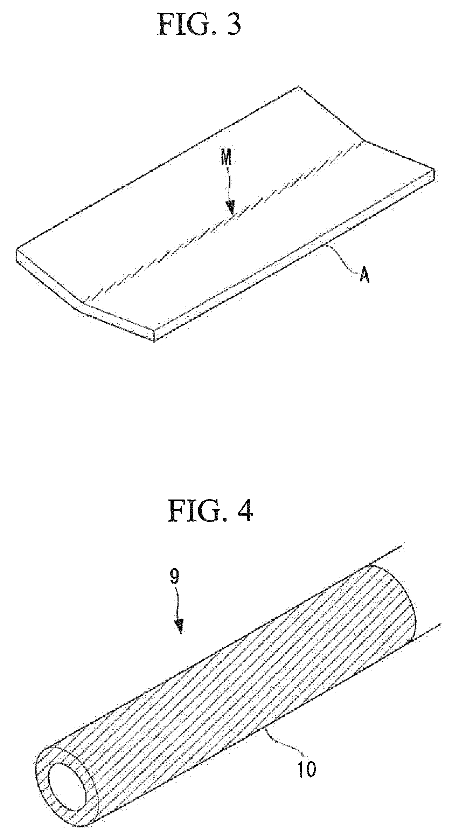

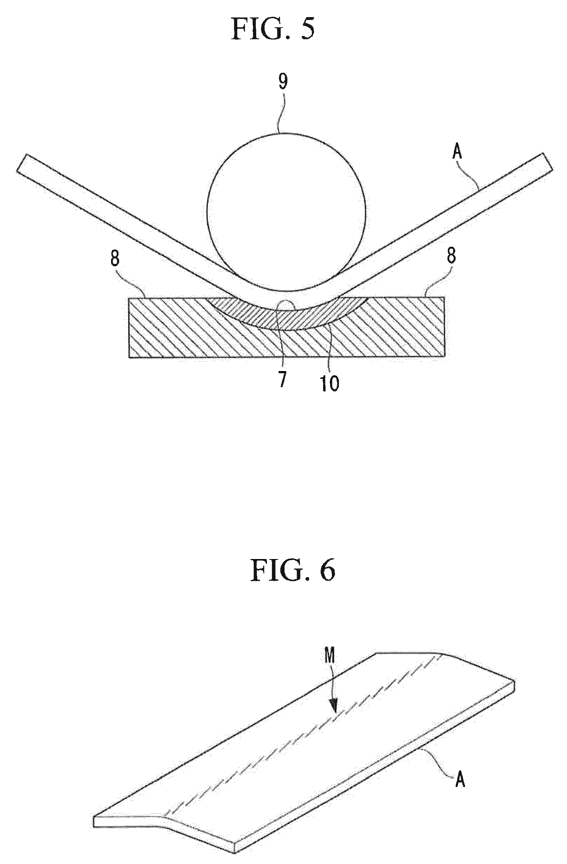

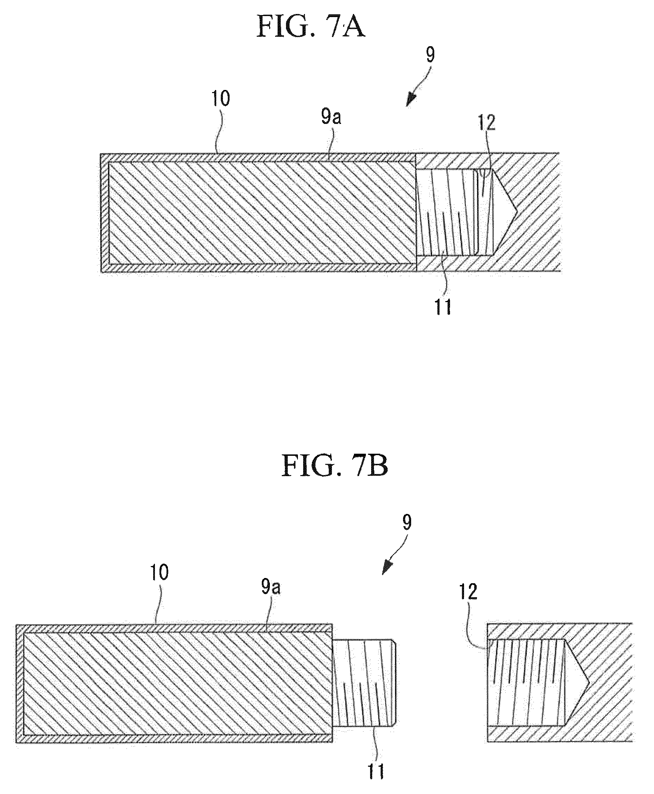

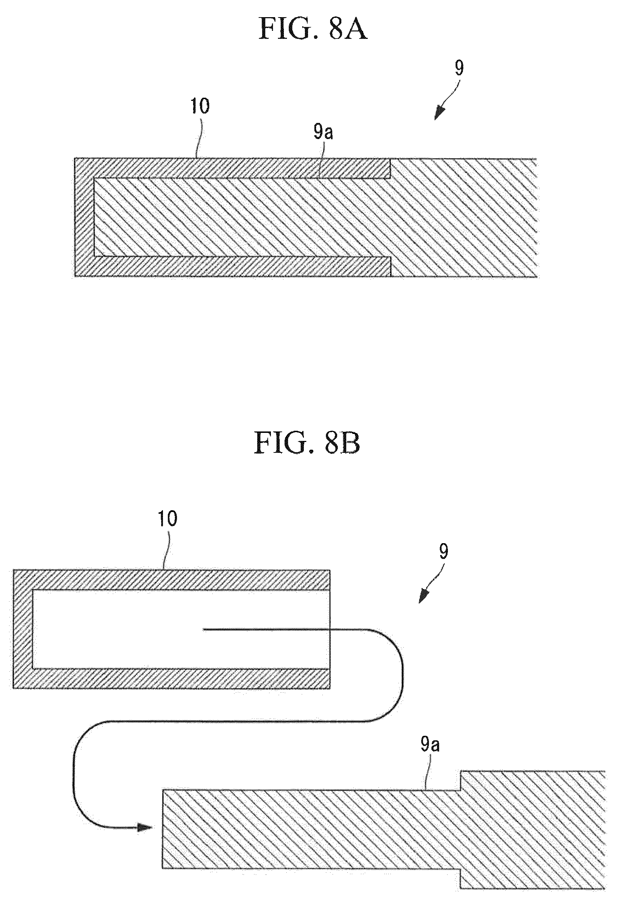

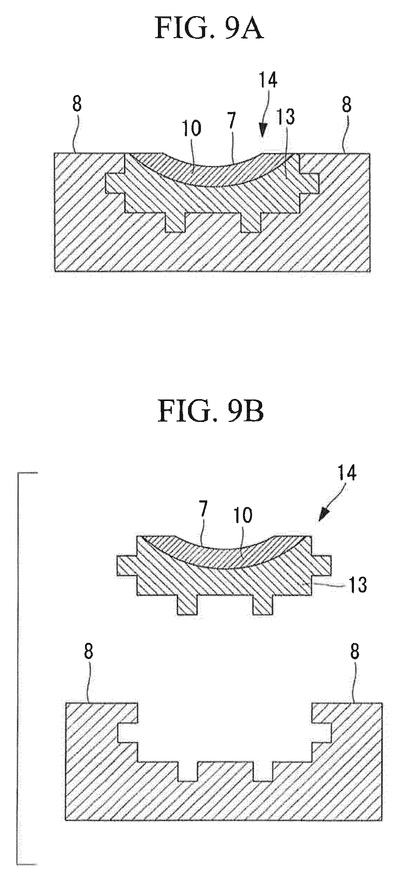

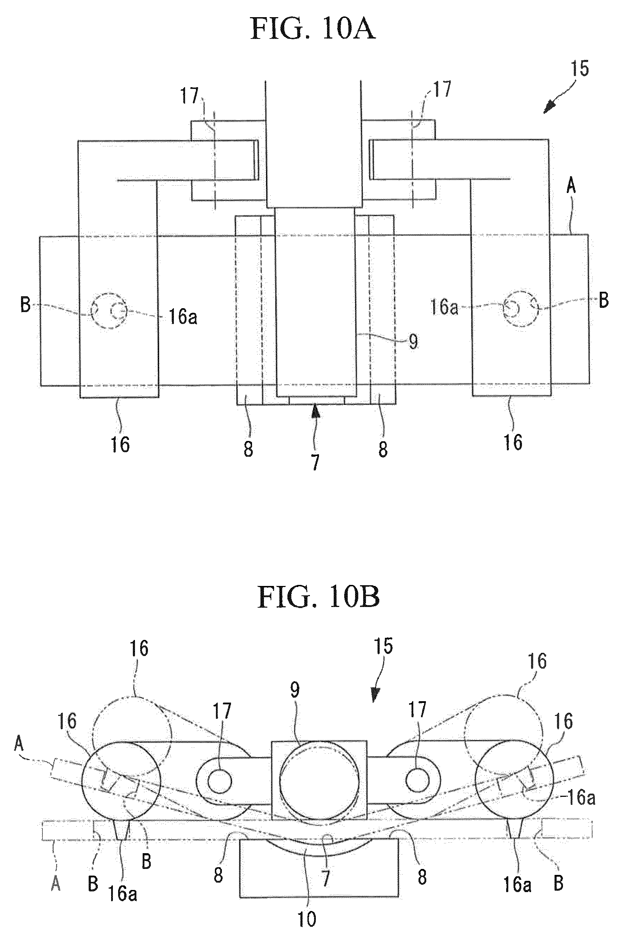

{Fig. 1} Fig. 1 is a side view illustrating a bending device according to an embodiment of the present invention.
{Fig. 2A} Fig. 2A is a perspective view illustrating a state where the distance between a pressing section and receiving sections of the bending device in Fig. 1 is increased.
{Fig. 2B} Fig. 2B is a perspective view illustrating a state where the distance between a pressing section and receiving sections of the bending device in Fig. 1 is reduced.
{Fig. 3} Fig. 3 is a perspective view illustrating a workpiece that has been bent by using the bending device in Fig. 1.
{Fig. 4} Fig. 4 is a perspective view illustrating a first modification of a marker retaining section of the bending device in Fig. 1.
{Fig. 5} Fig. 5 is a perspective view illustrating a second modification of the marker retaining section of the bending device in Fig. 1.
{Fig. 6} Fig. 6 is a perspective view illustrating a workpiece that has been bent by using the bending device in Fig. 5.
{Fig. 7A} Fig. 7A is a vertical sectional view illustrating the second modification of the marker retaining section of the bending device in Fig. 1, showing the marker retaining section in an attached state.
{Fig. 7B} Fig. 7B is a vertical sectional view illustrating the second modification of the marker retaining section of the bending device in Fig. 1, showing the marker retaining section in a detached state.
{Fig. 8A} Fig. 8A is a vertical sectional view illustrating a third modification of the marker retaining section of the bending device in Fig. 1, showing the marker retaining section in an attached state.
{Fig. 8B} Fig. 8B is a vertical sectional view illustrating the third modification of the marker retaining section of the bending device in Fig. 1, showing the marker retaining section in a detached state.
{Fig. 9A} Fig. 9A is a vertical sectional view illustrating a fourth modification of the marker retaining section of the bending device in Fig. 1, showing the marker retaining section in an attached state.
{Fig. 9B} Fig. 9B is a vertical sectional view illustrating the fourth modification of the marker retaining section of the bending device in Fig. 1, showing the marker retaining section in a detached state.
{Fig. 10A} Fig. 10A is a plan view illustrating a modification of the bending device in Fig. 1, showing a part thereof provided with a misalignment prevention mechanism.
{Fig. 10B} Fig. 10B is a front view illustrating the modification of the bending device in Fig. 1, showing the part thereof provided with the misalignment prevention mechanism.

### {Description of Embodiments}

A bending device 1 according to an embodiment of the present invention will be described below with reference to the drawings.

As shown in Fig. 1, the bending device 1 according to this embodiment includes two components 3 and 4 connected to each other in a pivotable manner about a pivot shaft 2. First ends of the two components 3 and 4 are respectively provided with grip sections 5 and 6 (operable sections) that are to be gripped by a physician with one hand. As shown in Figs. 2A and 2B, a second end of the first component 3 is provided with two planar receiving sections 8 spaced apart from each other with a groove 7, which substantially has a semicircular shape in cross section, interposed therebetween. A second end of the second component 4 is provided with a columnar pressing section 9, which is to be moved to a position where it engages with the groove 7 by pivoting the two components 3 and 4 relatively to each other.

As shown in Fig. 2A, the pressing section 9 of the bending device 1 according to this embodiment is provided with a marker retaining section 10. The marker retaining section 10 has a porous structure having multiple pores formed in the surface of the columnar pressing section 9. The pores of the porous structure retain powdery markers M therein. In this embodiment, the marker retaining section 10 is partially provided in a region that faces the groove 7 at the receiving section 8 side.

The powdery markers M used are preferably composed of a colored biocompatible material, such as (white-colored) biocompatible ceramic powder composed of, for example, calcium phosphate or magnesium phosphate.

A bending method using the bending device 1 according to this embodiment having the above-described configuration will be described below.

In order to bend a plate-like workpiece A by using the bending device 1 according to this embodiment, the plate-like workpiece A is set in a bridged manner between the two receiving sections 8, as shown in Fig. 2A (setting step). By gripping the grip sections 5 and 6, a pressing force acts on the pressing section 9 so that the workpiece A set on the receiving sections 8 is pressed into the space between the two receiving sections 8, as shown in Fig. 2B (pressing step).

Because the pressing section 9 is provided with the marker retaining section 10 and the markers M are retained within the marker retaining section 10, when the workpiece A is pressed as a result of the pressing section 9 being pressed against the surface of the workpiece A, the markers M retained in the marker retaining section 10 are transferred to the area of the workpiece A pressed by the pressing section 9. Thus, even when the bending is slight, the operator performing the bending process can easily recognize an already-bent area by simply visually-checking the markers M adhered to the surface of the workpiece A.

As a result, the bending device 1 and the bending method according to this embodiment are advantageous in that the workpiece A can be prevented from breaking as a result of performing a bending process again on an area that has already been bent once.

Furthermore, because the marker retaining section 10 is provided in the pressing section 9 in the bending device 1 and the bending method according to this embodiment, the markers M adhere to the recessed side of the bent workpiece A, as shown in Fig. 3. Therefore, the bent area can be readily recognized by visually checking the markers M on the recessed side of the workpiece A.

Furthermore, because the markers M used are composed of a biocompatible material in this embodiment, if the workpiece A used is to be implanted into the living body, like an osteosynthesis plate, the workpiece A can be implanted into the living body without having to remove the markers M adhered thereto after the bending process. Therefore, the operator, such as a physician, can omit the process for cleaning off the markers M.

Although the markers M to be adhered to the surface of the workpiece A are described as being powder in this embodiment, a liquid composed of a biocompatible material, such as a (black-colored) povidone-iodine liquid, may be used as an alternative.

Furthermore, although a colored biocompatible material, such as (white-colored) biocompatible ceramic powder composed of, for example, calcium phosphate or magnesium phosphate or a (black-colored) povidone-iodine liquid, is used, the markers M do not have to be biocompatible if the workpiece A is not intended to be implanted into the living body, like an osteosynthesis plate. Moreover, even if the workpiece A is intended to be implanted into the living body, the markers M do not have to be biocompatible so long as they can be cleaned off before the implantation. Furthermore, the markers M may be colorless if they are visually recognizable in the adhered state (based on, for example, a state in which they bulge out from the surface of the workpiece A).

Furthermore, in the bending device 1 according to this embodiment, the marker retaining section 10 is provided in a part of the columnar pressing section 9, in the circumferential direction thereof, at a position that faces the groove 7. Alternatively, as shown in Fig. 4, the marker retaining section 10 may be provided around the entire circumference of the pressing section 9.

Furthermore, although the marker retaining section 10 is provided in the pressing section 9 in this embodiment, the marker retaining section 10 may alternatively be provided at the receiving section 8 side, as shown in Fig. 5.

In the case where the marker retaining section 10 is provided at the receiving section 8 side, the marker retaining section 10 is preferably disposed at the bottom of the groove 7, as shown in Fig. 5. When the apex of the protruding side of the workpiece A bent as a result of the bending process comes into contact with the marker retaining section 10, the markers M adhere to the protruding side. Therefore, as shown in Fig. 6, the operator can visually check the markers M adhered to the protruding side of the workpiece A so as to readily recognize the bent area.

Furthermore, the pressing section 9 and the receiving section 8 side may both be provided with marker retaining sections 10. According to this configuration, the markers M adhere to the opposite surfaces of the workpiece A so that the operator can identify the bent area by looking at either one of the surfaces.

Furthermore, the marker retaining section 10 may be provided in a detachable manner in the pressing section 9 or at the receiving section 8 side. As a method for making the marker retaining section 10 detachable from the pressing section 9, a method of attaching the marker retaining section 10 by means of screws 11 and 12, as shown in Figs. 7A and 7B, or a method of fitting the marker retaining section 10, like a cap, around a core 9a disposed in the pressing section 9, as shown in Figs. 8A and 8B, may be employed.

Accordingly, without having to sterilize the marker retaining section 10, the bending process can be performed on a plurality of workpieces by replacing the marker retaining section 10. Moreover, when the markers M retained in the marker retaining section 10 are used up as a result of repeating the bending process, the markers M can be readily resupplied by simply replacing the marker retaining section 10.

In either case, the marker retaining section 10 disposed around the surface of the core 9a constituting the pressing section 9 increases the strength of the pressing section 9, thereby allowing for a stable bending process.

As a method of providing the marker retaining section 10 in a detachable manner at the receiving section 8 side, a unit 14 having the marker retaining section 10 fixed to a base 13 may be attached to the receiving sections 8 by being fitted therebetween, as shown in Figs. 9A and 9B.

Furthermore, the bending device 1 according to this embodiment may be equipped with a misalignment prevention mechanism 15 that prevents the pressing section 9 and the receiving sections 8 from being misaligned with the workpiece A during the bending process.

In a case where the workpiece A is a plate that has holes B at the opposite sides of an area where the workpiece A is to be bent, as shown in Fig. 10A, the misalignment prevention mechanism 15 may be constituted of, for example, handles 16, which have claws 16a to be fitted into the holes B, attached to the opposite sides of the pressing section 9 in a pivotable manner about axes 17 extending parallel to the longitudinal axis of the pressing section 9.

In the bending device 1 having such a misalignment prevention mechanism 15, when the pressing section 9 is moved closer toward the groove 7 between the receiving sections 8 by operating the grip sections 5 and 6 in a state where the workpiece A is set in a bridged manner between the receiving sections 8, as shown in Fig. 10B, the handles 16 disposed at the opposite sides of the pressing section 9 also move closer to the workpiece A. By bringing the pressing section 9 even closer to the groove 7, the claws 16a provided in the handles 16 become fitted into the holes B in the workpiece A, and the pressing section 9 subsequently comes into contact with the surface of the workpiece A.

Therefore, even when a pressing force is applied from the pressing section 9 to the workpiece A and the bending process is performed thereon, since the claws 16a of the handles 16 are fitted in the holes B in the workpiece A, the workpiece A is positioned so as to be prevented from being misaligned with the pressing section 9 and the receiving sections 8. Moreover, when the bending process is performed, the positions of the holes B change as a result of the bent workpiece A. However, since the handles 16 pivot about the axes 17 in correspondence with this positional change, the positions of the claws 16a also change so that the claws 16a are maintained in the fitted state in the holes B. Consequently, the workpiece A is supported without being misaligned with the pressing section 9 and the receiving sections 8 even during the bending process.

### {Reference Signs List}

- A: workpiece
- M: markers
- 1: bending device
- 5, 6: grip sections (operable sections)
- 8: receiving sections
- 9: pressing section
- 10: marker retaining section

## Claims

1. A bending method comprising:
a setting step for setting a plate-like workpiece in a bridged manner between two receiving sections disposed with a distance therebetween; and
a pressing step for causing a pressing force to act on a pressing section so as to press the workpiece set between the receiving sections into a space between the receiving sections,
wherein the pressing step includes causing a marker to adhere to a pressed area of whichever one of surfaces of the workpiece, the marker indicating that the area has been pressed.

2. The bending method according to Claim 1,
wherein the pressing step includes disposing the marker at the pressing section side of the workpiece and causing the marker to adhere to the surface of the workpiece by means of the pressing force.

3. The bending method according to Claim 2,
wherein the pressing step includes pressing against the workpiece by using the pressing section, which retains the marker on a surface thereof at the workpiece side.

4. The bending method according to Claim 1,
wherein the pressing step includes disposing the marker at the receiving section side of the workpiece and causing the marker to adhere to the surface of the workpiece by means of the pressing force.

5. The bending method according to any one of Claims 1 to 4,
wherein the workpiece is an osteosynthesis plate, and
wherein the marker is composed of a biocompatible material.

6. A bending device comprising:
two receiving sections disposed with a distance therebetween;
a pressing section that presses a plate-like workpiece, which is supported in a bridged manner between the receiving sections, into a space between the receiving sections;
an operable section that applies an external force for causing the pressing section to generate a pressing force; and
a marker retaining section that is attached to at least one of the pressing section and the receiving section side and that retains a marker to be adhered to a pressed area of whichever one of surfaces of the workpiece by means of the pressing force.

7. The bending device according to Claim 6,
wherein the marker retaining section is detachably attached to at least one of the pressing section and the receiving section side.

8. The bending device according to Claim 6 or 7,
wherein the marker is formed of powder or liquid,
wherein the marker retaining section has a porous structure that is capable of retaining the marker within pores.

9. The bending device according to any one of Claims 6 to 8,
wherein the marker retaining section is attached to a surface at the receiving-section side of the pressing section.

10. The bending device according to any one of Claims 6 to 8,
wherein the marker retaining section is attached between the two receiving sections.

11. The bending device according to any one of Claims 6 to 10,
wherein the workpiece is an osteosynthesis plate, and
wherein the marker is composed of a biocompatible material.
